# EUROPÄISCHE PATENTSCHRIFT

(11) **EP 2 413 972 B1**
(45) Veröffentlichungstag und Bekanntmachung des Hinweises auf die Patenterteilung: **10.03.2021**
(21) Anmeldenummer: 10712072.7
(22) Anmeldetag: 31.03.2010
(51) Int. Cl.: A61K 49/00, A61L 15/42, A61Q 19/00, A61P 17/00, A61K 47/61

(54) **VERKNÜPFUNGSPRODUKTE AMINIERTER POLYSACCHARIDE**
BONDING PRODUCTS OF AMINATED POLYSACCHARIDES
PRODUITS DE LIAISON DE POLYSACCHARIDES AMINÉS

(30) Priorität: 31.03.2009 DE 102009015085; 07.12.2009 WO PCT/EP2009/008718
(43) Veröffentlichungstag der Anmeldung: 08.02.2012
(73) Patentinhaber: B. Braun Melsungen AG, 34212 Melsungen (DE)
(72) Erfinder: MEIER, Bernd Horst, 64285 Darmstadt (DE); JANKOWIAK-MEIER, Iris, 64285 Darmstadt (DE); MEIER, Nele, 64285 Darmstadt (DE); MEIER, Clara, 64285 Darmstadt (DE)
(74) Vertreter: Prüfer & Partner mbB Patentanwälte · Rechtsanwälte
(86) Internationale Anmeldenummer: PCT/EP2010/054295
(87) Internationale Veröffentlichungsnummer: WO 2010/136242

(56) Entgegenhaltungen:
- EP-A2- 0 051 354
- WO-A1-96/02260
- WO-A1-03/074087
- WO-A1-2004/024761
- WO-A1-2007/122269
- WO-A2-02/080979
- WO-A2-2007/101698
- BAJPAI A K ET AL: "Dynamics of controlled release of heparin from swellable crosslinked starch microspheres" JOURNAL OF MATERIALS SCIENCE: MATERIALS IN MEDICINE, KLUWER ACADEMIC PUBLISHERS, BO LNKD- DOI:10.1007/S10856-007-3020-Y, Bd. 18, Nr. 8, 5. Mai 2007 (2007-05-05), Seiten 1613-1621, XP019529206 ISSN: 1573-4838
- A. V. Demchenko: "Handbook of Chemical Glycosylation: Advances in Stereoselectivity and Therapeutic Relevance", 2008, Wiley-VCH ISBN: 978-3-527-31780-6 pages 1-27,

## Beschreibung

Die Erfindung betrifft ein Verknüpfungsprodukt umfassend mindestens die Polysaccharide T1 und T2, wobei die Monosaccharide, aus denen die Polysaccharide T1 und T2 aufgebaut sind, vollständig alpha-1,4-glycosidisch miteinander verknüpft sind und mindestens eines der Polysaccharide T1 und/oder T2 wenigstens eine Aminogruppe aufweist und T1 und T2 durch mindestens einen Linker Z miteinander chemisch kovalent verbunden sind.

Darüber hinaus betrifft die Verbindung pharmazeutische Zubereitungen, die ein solches Verknüpfungsprodukt umfassen, wobei die pharmazeutische Zubereitung zur Verwendung in der Prophylaxe von Verwachsungen und Narbenbildungen sowie zur Stillung von Blutungen oder als Synovialflüssigkeit verwendet werden kann. Darüber hinaus sind die pharmazeutischen Zubereitungen zur Verwendung in der Therapie und Prophylaxe der Wundheilung vorgesehen.

Darüber hinaus betrifft die Erfindung die Verwendung des Verknüpfungsprodukts als Wundabdeckung oder als Implantat. Weiterhin betrifft die Erfindung ein Verfahren zur Herstellung des Verknüpfungsprodukts.

Im medizinischen Bereich haben Kunststoffe in den vielfältigsten Gebieten Einzug gehalten. Kunststoffe werden beispielsweise eingesetzt als Implantate, Nahtmaterial, Gefäßprothesen, Gefäßkatheder oder auch als Isolationsmaterial für elektrische Leiter. Die verwendeten Kunststoffe stehen dabei häufig in einem steten Kontakt zu den Geweben des menschlichen Körpers. Bereits kurz nach der Implantation beginnt allerdings eine komplexe Auseinandersetzung der körperlichen Abwehrsysteme des Implantatträgers mit dem Fremdkörper. Die Auseinandersetzung kann zur Abstoßung des implantierten Fremdmaterials, im ungünstigen Fall zu schweren Entzündungsreaktionen führen. Die Implantate können darüber hinaus bakteriell infiziert werden, was zur Ausstreuung der Bakterien über das Blut bis hin zur lebensbedrohlichen Sepsis führen kann. Diese Komplikationen machen es notwendig, manche Implantate nur so kurz wie möglich im Körper zu belassen. Die Probleme führen zu der Verwendung von Kunststoffen, die nur geringe Angriffsflächen für die immunologische Auseinandersetzung bieten. Eine andere Schwierigkeit stellt die Aktivierung der Blutgerinnung durch jene Implantate dar, die Kontakt zum zirkulierenden Blut haben, wie Gefäßprothesen, Stents, oder zentralvenöse Katheder. Durch Kontaktaktivierung an Implantaten entstandene Gerinsel können Katheder und Stents verstopfen und damit unbrauchbar machen. Auch können Blutgerinsel sehr häufig Ausgangspunkt für eine weitere bakterielle Besiedlung sein. Diese Komplikationen versucht man durch die Beschichtung der dem Blut zugewandten Oberfläche der Implantate mit gerinnungshemmenden Substanzen, wie Heparin, zu begegnen. Einige Implantate, wie z.B. Stents machen es notwendig, diese Bildung von Blutgerinseln durch eine Behinderung der Blutgerinnung durch die zusätzliche Einnahme von gerinnungshemmenden Medikamenten, wie Phenprocoumen, Clopidogrel, Acetylsalicylsäure oder Heparine zu verhindern. Diese systemische Antikoagulation ist ebenfalls mit einem erheblichen Risiko einer Blutung verbunden. Bei einer großen Anzahl von zentralarteriellen und zentralvenösen Kathedern werden formbare Kunststoffverbindungen, wie Polyvinylchlorid oder Polyurethanverbindungen verwendet. Bei diesen Kathedern mit dauerndem Kontakt zu dem zirkulierenden Blut wird durch eine Beschichtung mit gerinnungshemmenden Substanzen und/oder mit einer entsprechenden Glättung der Oberfläche einer Haftung von Gerinnungskörpern entgegengewirkt. Bei den Beschichtungen dieser Kunststoffe mit Heparinen sind verschiedene Techniken von der ladungsabhängigen Anlagerung der stark negativ geladenen Heparinmoleküle bis zur chemisch kovalenten Bindung unter Ausbildung von Linkern denkbar. Bei allen Beschichtungen mit Heparin sind solche Verfahren zu bevorzugen, die den gerinnungshemmenden Teil des Heparinmoleküls freihalten. Die ideale Kopplung über die terminale Aldehydgruppe des Heparinmoleküls ist jedoch wegen des Fehlens geeigneter funktioneller Gruppen im Kathedermaterial nur mit erhöhtem Aufwand durchführbar. Kunststoffe sind im Wesentlichen biologisch weitgehend inert. Implantate aus diesen Kunststoffen werden eher als Fremdkörper erkannt und abgestoßen als vom Körper epithelialisiert und körpergerecht umgesetzt, wie z.B. bei Implantaten biologischer Herkunft.

WO 2004/024761 beschreibt ein Hydroxyalkylstärke-Erythrpoetin-Konjugat (HAS-EPO-Konjugat), das ein oder mehrere Moleküle HAS umfasst, wobei jedes HAS-Molekül an das EPO via einer Carbohydridgruppe oder einem Thioether geknüpft ist.

WO 02/080979 betrifft ein HAS-Wirkstoff-Konjugat, erhältlich durch Verfahren zur Herstellung eines kovalenten HAS-Wirkstoff-Konjugats, bei dem man HAS und einen Wirkstoff in einem Reaktionsmedium miteinander umsetzt, wobei das Reaktionsmedium Wasser oder ein Gemisch von Wasser mit organischem Lösungsmittel, welches mindestens 10 Gew.-% Wasser umfasst, ist.

WO 2007/101698 offenbart Konjugate aus Hydroxyalkylstärke (HAS) und verschiedener pharmazeutisch wirksamer Peptide, die unter anderem durch die Verwendung von aminierter HAS durch reduktive Aminierung zugänglich sind.

Aus der WO 03/074087 sind dem Fachmann Hydroxyalkylstärke-Protein-Konjugate bekannt, bei denen die bindende Wechselwirkung zwischen dem Hydroxyalkylstärkemolekül und dem Protein auf einer kovalenten Bindung beruht, welche das Ergebnis einer Kopplungsreaktion zwischen (i) der endständigen Aldehydgruppe oder aus dieser Aldehydgruppe durch chemische Umsetzung hervorgegangenen funktionellen Gruppe des Hydroxyalkylstärkemoleküls und (ii) einer mit dieser Aldehydgruppe oder daraus hervorgegangenen funktionellen Gruppe des Hydroxyalkylstärkemoleküls reaktionsfähigen funktionellen Gruppe des Proteins ist, wobei die bei der Kopplungsreaktion unmittelbar resultierende Bindung gegebenenfalls durch eine weitere Reaktion zur oben genannten kovalenten Bindung modifiziert sein kann.

WO 2007/122269 betrifft ein Verknüpfungsprodukt aus m Molekülen eines Polysaccharids X mit einem Polysaccharid A, wobei das Polysaccharid A aus der Gruppe der Chitine oder Chitosane ausgewählt ist, m eine ganze Zahl zwischen 1 und 10.000 ist und die Verknüpfung zwischen X und A eine Amin- und/oder Amidbindung ist.

WO 96/02260 beschreibt die Verwendung von Chitosan in Kombination mit einem ersten Polysaccharid, das ausgewählt ist aus Heparin, Heparinsulfat und Dextransulfat, zum Herstellen eines Mittels, das die Heilung von Hautwunden beschleunigt, stimuliert und/oder fördert, wobei das Polysaccharid gegebenenfalls an dem Chitosan immobilisiert ist.

In der US 2005/828800 wird die reduktive Aminierung von Hydroxyalkylcellulose Verbindungen beschrieben. Die dort dargestellten Verbindungen sind Feststoffe, bestehend aus beta-glycosidisch verknüpften Anhydroglcuoseeinheiten. Diese Verbindungen sind für die erfindungsgemäßen Zwecke aufgrund ihrer physikochemischen Eigenschaften völlig ungeeignet. Eine spezifische Einbindung weiterer Substituenten über die eingeführten Aminogruppen wird nicht beschrieben. Vielmehr weisen die reduktiv aminierten beta-glycosidisch verbundenen Hydroxyalkylcellulosen große Ähnlichkeit mit den Chitosanen auf. Aminierte Cellulosen sind im Vergleich zu Chitosan verzweigt und ihre Glucosaminmonomere sind nicht an den Aminogruppen acetyliert, wie es beispielsweise bei Chitosan in bis zu 40% der Fall ist. Beide Verbindungen sind jedoch wasserunlösliche Poly-beta-1,4-glucosamine. Höhere Säuger können weder beta-glycosidisch verknüpfte Chitosane, noch aminierte Hydroxyalkylcellulosen durch körpereigene Enzyme abbauen. Es ist davon auszugehen, dass die aminierten Hydroxyalkylcellulosen ein deutlich höheres allergisches Potential aufweisen, als Chitosanverbindungen. Andere elastisch formbare Polymere werden in der Chirurgie als Polymethylmethacrylate, beispielsweise als Knochenzement mit den im Vorigen beschriebenen Risiken der allergischen Reaktion bis hin zum allergischen Schock eingesetzt.

Es bestand daher ein Bedarf, geeignete Verbindungen zur Verfügung zu stellen, die die im Stand der Technik genannten Probleme lösen. Insbesondere bestand die Aufgabe der vorliegenden Erfindung darin, biologisch abbaubare Verbindungen zur Verfügung zu stellen, die als polymere Grundstoffe oder Additive, vorzugsweise in medizinischen Artikeln eingesetzt werden können und die darüber hinaus einfach mit Arzneimittelwirkstoffen und/oder Fluoreszenzmarkern verknüpft werden können.

Überraschend wurde nunmehr gefunden, dass die im Stand der Technik vorhandenen Probleme gelöst werden können durch ein Verknüpfungsprodukt, welches mindestens zwei Polysaccharide umfasst, bei denen die Monosaccharideinheiten alpha-1,4-glycosidisch miteinander verknüpft sind und die mindestens eine Aminogruppe aufweisen.

Gegenstand der vorliegenden Erfindung ist daher ein Verknüpfungsprodukt gemäß Anspruch 1. Bevorzugte Ausführungsformen werden in den Unteransprüchen beschrieben.

Das erfindungsgemäße Verknüpfungsprodukt umfasst mindestens zwei Polysaccharide T1 und T2, die aus Monosacchariden aufgebaut sind, die vollständig alpha-1,4-glycosidisch miteinander verknüpft sind. Darüber hinaus trägt mindestens eines der Polysaccharide, die in dem Verknüpfungsprodukt vorliegen wenigstens eine Aminogruppe.

Die aminierten Polysaccharide stellen ideale Ausgangsverbindungen für weitere Verknüpfungsreaktionen mit beispielsweise Arzneimittelwirkstoffen oder auch für die Verknüpfung zwischen den Polysacchariden T1 und T2 dar.

Das erfindungsgemäße Verknüpfungsprodukt weist als Polysaccharide T1 und T2 solche auf, welche unabhängig voneinander ausgewählt sind aus der Gruppe, bestehend aus aminierter Hydroxyethylstärke, aminierter Carboxymethylstärke, aminierter Carboxymethylstärke, aminierter Hydroxyethyl-Carboxymethylstärke und aminierter Hydroxyalkylstärke.

In einer weiteren bevorzugten Ausführungsform sind die Polysaccharide T1 und T2 des erfindungsgemäßen Verknüpfungsprodukts unterschiedlich voneinander.

In einer weiteren bevorzugten Ausführungsform sind die aminierten Polysaccharide T1 und/oder T2 bei 20 °C wasserlöslich, vorzugsweise weisen T1 und/oder T2 eine Wasserlöslichkeit bei 20 °C von mindestens 1 g/l, vorzugsweise 10 g/l, insbesondere 50 g/l auf.

Die Polysaccharide T1 und/oder T2 weisen wenigstens eine Aminogruppe auf. In einer bevorzugten Ausführungsform weist sowohl das Polysaccharid T1 als auch das Polysaccharid T2 wenigstens eine Aminogruppe auf.

Als Aminogruppen können die Polysaccharide T1 und/oder T2 sowohl primäre, sekundäre als auch tertiäre Aminogruppen aufweisen, jedoch zeigen die Polysaccharide T1 und/oder T2 mindestens eine -NH₂-Gruppe auf.

Die Einführung von Aminogruppen in Polysaccharide ist dem Fachmann geläufig. Die Aminogruppen werden bevorzugt durch reduktive Aminierung der Polysaccharide T1 und/oder T2 eingeführt. Die Polysaccharide T1 und/oder T2 weisen somit bevorzugt Aminogruppen auf, die durch reduktive Aminierung in die Polysaccharide T1 und/oder T2 eingeführt wurden. Solche Polysaccharide T1 und/oder T2 sind daran erkennbar, dass die Aldehydgruppen der Polysaccharide T1 oder T2 in Aminogruppen (-NH₂-Gruppen) überführt wurden.

Die Polysaccharide T1 und T2, aus denen das Verknüpfungsprodukt aufgebaut ist, weisen Monosaccharide auf, die vollständig alpha-1,4-glycosidisch miteinander verknüpft sind. Die alpha-1,4-glycosidische Verknüpfung der Monosaccharide trägt erheblich zu einer besseren biologischen Abbaubarkeit der Polysaccharide bei.

Je nach Einsatzgebiet kann das Molekulargewicht der Polysaccharide T1 und T2 variieren. Bevorzugt liegt das mittlere Molekulargewicht der Polysaccharide T1 und/oder T2 in einem Bereich von 20.000 bis 800.000 Dalton, vorzugsweise 25.000 bis 500.000 Dalton, insbesondere von 30.000 bis 200.000 Dalton.

Als besonders bevorzugte Polysaccharide T1 und/oder T2 hat sich modifizierte Stärke, insbesondere Hydroxyethylstärke, mit einem Substitutionsgrad DS zwischen 0,2 und 0,8, vorzugsweise zwischen 0,3 und 0,8 herausgestellt, wobei die modifizierte Stärke bzw. die Hydroxyethylstärke aminiert vorliegt.

Der Substitutionsgrad DS ist definiert als das Verhältnis der Gesamtzahl der substituierten Monomereinheiten zur Gesamtzahl der Monomereinheiten.

Die erfindungsgemäßen Verknüpfungsprodukte sind mit Arzneimittelstoffen und/ oder Fluoreszenzmarkern verknüpft .

Insbesondere für medizinische Produkte hat sich der Einsatz von Arzneimittelwirkstoffen A als vorteilhafte Ausrüstung der Verknüpfungsprodukte gezeigt.

Daher handelt es sich bei dem Arzneimittelwirkstoff A um Heparin, insbesondere Heparin mit weniger als 6 Saccharideinheiten.

Der Arzneistoff A wird bevorzugt durch reduktive Aminierung mit den Polysacchariden T1 und/oder T2, die bereits durch Z miteinander verknüpft sein können, verknüpft.

Die Polysaccharide T1 und/oder T2 weisen bevorzugt m-Gruppen-(L-A) auf, wobei m eine ganze Zahl ist, die mindestens 1, vorzugsweise 1 bis 1000, insbesondere 1 bis 100, weiter bevorzugt 2 bis 100 und insbesondere 3 bis 20 ist.

Die Fluoreszenzmarker sind bevorzugt ausgewählt aus der Gruppe bestehend aus Fluoresceinisothiocyanat (FITC), Phycoerythrin, Rhodamid und 2- Aminopyridin.

Neben rein arzneilich wirkenden Stoffen können auch Fluoreszenzmarker z.B. Fluoresceinisothiocyanat in Verbindung mit den Polysacchariden T1 und/oder T2 eingesetzt werden. Die Markierung mit Fluoreszenzmarkern kann im medizinischen Bereich dazu dienen, diese Verknüpfungsprodukte spezifisch im Körper sichtbar zu machen. Der Einsatz der erfindungsgemäßen Verknüpfungsprodukte im kosmetischen Bereich kann beispielsweise dort zu unter UV-lichtstrahlenden Haargelen, Haarfestigern oder Tönungsmitteln führen.

Die Polysaccharide T1 und T2 sind durch mindestens einen Linker Z miteinander chemisch kovalent verbunden. In einer bevorzugten Ausgestaltung der vorliegenden Erfindung ist der Linker Z eine funktionelle Gruppe, ausgewählt aus Carbonsäureester, Carbonsäureamide, Urethan, Ether und Amin oder umfasst zumindest eine solche funktionelle Gruppe. Insbesondere bevorzugt ist die kovalente chemische Bindung zwischen T1 und T2 über den Linker Z reversibel, also ohne weiteres, beispielsweise enzymatisch, wieder spaltbar.

Der zweite Linker L, durch den T1 und/oder T2 mit A kovalent verknüpft ist, entspricht in seiner Funktion und Ausgestaltung ebenfalls dem ersten Linker Z. Für den Linker L ist es besonders vorteilhaft, wenn dieser ohne weiteres, beispielsweise enzymatisch, wieder spaltbar ist, wodurch es zur Freisetzung des Arzneimittelstoffs und/oder des Fluoreszenzmarkers kommt. Die Bildung des Linkers Z oder L kann mit Hilfe der im Stand der Technik beschriebenen Methoden zur Bildung von Carbonsäureestern, Carbonsäureamide, Urethanen, Ethern und Aminen erfolgen.

Weisen sowohl T1 als auch T2 Aminogruppen auf, erfolgt die Verknüpfung vorzugsweise durch aliphatische Dialdehyde, beispielsweise Glutaraledyd.

Als besonders vorteilhaft haben sich Verknüpfungsprodukte herausgestellt, bei denen die Verknüpfung von T1 und T2 über eine reduktive Aminierung erfolgt. Die erfindungsgemäßen Verknüpfungsprodukte sind somit erhältlich durch reduktive Aminierung eines freie Aminogruppen (-NH₂) aufweisenden Polysaccharids T1 mit einem Polysaccharid T2, das mindestens eine Aldehyd- oder Ketogruppe aufweist und wobei das Polysaccharid T1 und/oder T2 mit m Resten -(L-A) verknüpft ist.

Das Aminogruppen aufweisende Polysaccharid T1 ist hierbei ausgewählt aus der Gruppe bestehend aus aminierter Stärke, aminierter Hydroxyalkylstärke, aminierter Hydroxyalkylcarboxyalkylstärke und aminierter Carboxyalkylstärke. Insbesondere bevorzugt ist aminierte Hydroxyalkylstärke, die beispielsweise selbst durch reduktive Aminierung erhalten werden kann.

In einer bevorzugten Ausführungsform ist das erfindungsgemäße Verknüpfungsprodukt erhältlich durch reduktive Aminierung eines freie Aminogruppen (-NH₂) aufweisenden Polysaccharids T1 mit einem Polysaccharid T2, welches mindestens eine Aldehyd- oder Ketogruppe aufweisen und wobei das Polysaccharid T1 und/oder das Polysaccharid T2 mit m-Resten (L-A) verknüpft ist.

Insbesondere bevorzugt ist das Aminogruppen aufweisende Polysaccharid T1 ausgewählt aus der Gruppe bestehend aus aminierter Stärke, aminierter Hydroxyethylstärke, aminierter Hydroxyalkylstärke, aminierter Hydroxyalkylcarboxyalkylstärke und aminierter Carboxyalkylstärke. Der Arzneimittelwirkstoff A ist Heparin.

In einer besonders bevorzugten Ausführungsform ist das erfindungsgemäße Verknüpfungsprodukt derart ausgestaltet, dass A Heparin, m mindestens 1 und das Polysaccharid T1 und/oder T2 eine Hydroxyethylstärke ist und der Linker L eine -NH-Gruppe darstellt.

In einer bevorzugten Ausführungsform ist der Linker L eine funktionelle Gruppe, ausgewählt aus Carbonsäureester, Carbonsäureamide, Urethan, Ether und Amin oder umfasst eine solche Gruppe.

Je nach Anwendungsgebiet kann die Verknüpfung der Polysaccharide T1 und T2 über die Linker Z auch zu größeren Clustern verknüpft werden. Erfindungsgemäß kann das Verhältnis dieser Verknüpfungsreaktion durch geeignete Modifikation des verwendeten Verfahrens beeinflusst werden. Dies kann beispielsweise und am einfachsten durch Veränderung des Verhältnisses der eingesetzten Polysaccharide T1 und T2 sowie der eingesetzten Verknüpfungssubstrate als auch durch die Modifikation des Molgewichts der eingesetzten Polysaccharide T1 und T2 erfolgen. Darüber hinaus beeinflussen auch Reaktionsbedingungen, wie Temperaturdruck und Katalysatoren das Verhältnis der beiden Reaktanden. Dies ist jedoch dem Fachmann geläufig. In einer bevorzugten Ausführungsform umfasst das Verknüpfungsprodukt neben den Polysacchariden T1 und T2 noch weitere Polysaccharide. In einer besonders bevorzugten Ausführungsform umfasst das Verknüpfungsprodukt jedoch ausschließlich die Polysaccharide T1 und T2, die gegebenenfalls mit m-Gruppen, (L-A) verknüpft sind.

Das Verknüpfungsprodukt der vorliegenden Erfindung kann als Flüssigkeit, Hydrogel, Film oder Feststoff vorliegen. In einer bevorzugten Ausführungsform liegt das Verknüpfungsprodukt als polymerer Feststoff vor und hat vorzugsweise ein mittleres Molekulargewicht von mindestens 50000 Dalton, bevorzugt mindestens 100000 Dalton, insbesondere 120 bis 2000000 Dalton.

Das Verknüpfungsprodukt der vorliegenden Erfindung ist erhältlich durch Verknüpfen von mindestens den Polysacchariden T1 und T2, wobei die Monosaccharide, aus denen die Polysaccharide T1 und T2 aufgebaut sind, vollständig alpha-1,4-glycosidisch miteinander verknüpft sind und mindestens eines der Polysaccharide T1 und/oder T2 wenigstens eine Aminogruppe aufweist, durch mindestens einen Linker Z, der T1 und T2 chemisch kovalent miteinander verknüpft und wobei T1 und/oder T2 m-Gruppen (L-A) trägt.

Ein weiterer Gegenstand der vorliegenden Erfindung ist eine pharmazeutische Zubereitung, die das erfindungsgemäße Verknüpfungsprodukt umfasst.

Die pharmazeutische Zubereitung kann dabei zur Prophylaxe von Verwachsungen und Narbenbildungen verwendet werden. Es hat sich überraschend gezeigt, dass die Applikation des erfindungsgemäßen Verknüpfungsproduktes in Form eines Hydrogels die Narbenbildung und insbesondere Verwachsungen vermeiden kann. Dies ist insbesondere bei der postoperativen Versorgung von Patienten von großer Bedeutung.

Darüber hinaus können die pharmazeutischen Zubereitungen der vorliegenden Erfindung zur Stillung von Blutungen verwendet werden oder die pharmazeutische Zubereitung wird als Synovialflüssigkeit verwendet.

Es hat sich zudem überraschend gezeigt, dass die Verknüpfungsprodukte der vorliegenden Erfindung in der Therapie und Prophylaxe der Wundheilung verwendet werden können. So ist es bevorzugt, dass die Verknüpfungsprodukte der vorliegenden Erfindung für Wundabdeckungen verwendet werden. Das Produkt kann dort als Hydrogel, Feststoff oder Flüssigkeit in die Wundauflage implementiert werden. Darüber hinaus werden die erfindungsgemäßen Verknüpfungsprodukte als Implantate verwendet. Insbesondere die Ausrüstung der erfindungsgemäßen Verknüpfungsprodukte mit Heparin hat gezeigt, dass diese insbesondere bei medizinischen Artikeln, die mit dem Gewebe oder Körperflüssigkeiten in Kontakt stehen, hervorragende Eigenschaften zeigen. Die erfindungsgemäßen Produkte können auch lediglich als Additiv zu Implantaten oder medizinischen Artikeln zugesetzt werden.

Ein weiterer Gegenstand der vorliegenden Erfindung ist ein Verfahren zur Herstellung des erfindungsgemäßen Verknüpfungsprodukts.

Das Verfahren zur Herstellung des erfindungsgemäßen Verknüpfungsprodukts erfolgt durch Verknüpfung mindestens eines Polysaccharids T1 mit mindestens einem Polysaccharid T2 unter Ausbildung des Linkers Z mit den T1 und T2 kovalent verknüpft sind und wobei T1 und/oder T2 m-Gruppen (L-A) trägt, wobei
- A ein Arzneimittelwirkstoff und/oder ein Fluoreszenzmarker,
- L ein zweiter Linker, durch den T1 und/oder T2 mit A kovalent verknüpft ist und m eine ganze Zahl ist, die mindestens 1 ist.

Für T1, T2, A, Z, L und m gelten die zuvor aufgeführten bevorzugten Ausgestaltungen.

In einer bevorzugten Ausführungsform des erfindungsgemäßen Verfahrens werden die Polysaccharide T1 und/oder T2 zunächst mit dem Arzneimittelwirkstoff A verknüpft und anschließend die Ausbildung des Linkers Z durchgeführt.

In einer besonders bevorzugten Ausführungsform des vorliegenden erfindungsgemäßen Verfahrens erfolgt das Verfahren durch die folgenden Schritte:
a) reduktive Aminierung einer Hydroxyethylstärke,
b) Verknüpfung der in Schritt a) erhaltenen aminierten Hydroxyethylstärke mit Heparin durch reduktive Aminierung, und
c) Verknüpfen des in Schritt b) erhaltenen Produkts mit Hydroxyethylstärke unter Ausbildung eines Linkers Z.

Besonders bevorzugt erfolgt die Verknüpfung der Polysaccharide T1 und/oder T2 wie zuvor definiert über den zweiten Linker L mit dem Arzneimittelwirkstoff A. Für die Ausbildung des Linkers L mit dem Arzneimittelwirkstoff Adem Heparin werden bevorzugt bifunktionelle und trifunktionelle Moleküle eingesetzt, die identische oder unterschiedliche funktionelle Gruppen aufweisen, die mit den funktionellen Gruppen des Heparins als auch den funktionellen Gruppen des/der Polysaccharids reagieren können. Hierbei kann es jedoch zu unerwünschten Verbindungen der Heparinmoleküle und Polysaccharidmoleküle untereinander (Vernetzung) kommen. Diese Reaktionsprodukte konkurrieren mit den erwünschten Verbindungen zwischen Heparin und Polysaccharid T1 und/oder T2. Deshalb sind besonders bipolyfunktionelle Moleküle mit unterschiedlichen funktionellen Gruppen geeignet, die einerseits mit einer funktionellen Gruppe reagieren, die nur auf dem Heparin, andererseits mit einer funktionellen Gruppe reagieren, die im Polysaccharid vorhanden ist. Dies setzt in der Regel eine entsprechende chemische Veränderung auf der Seite des Polysaccharids (T1 und/oder T2), seltener des Heparins voraus. Die Ausbeute an den erfindungsgemäßen Verknüpfungsprodukten kann durch die Immobilisation des Heparins an geeignete Anlagerungskörper deutlich erhöht werden.

Für die bei Carboxymethylstärken vorhandenen Carboxylgruppen kommen Verbindungen als Linker zur Anwendung, die ausgewählt sind aus der Gruppe der Diepoxyalkane, die vorzugsweise 4 bis 16 Kohlenstoffatome aufweisen, insbesondere 1,2,3,4-Diepoxybutan, 1,2,7,8-Diepoxoctan, oder alternativ Glutaraldehyd. Diepoxyalkane bilden unter sauren pH-Werten, vorzugsweise im Bereich von 2 bis 4, Esterbindungen, während sie i alkalischen pH-Bereich (pH > 10) Etherbindungen ausbilden. Glutaraldehyd reagiert bevorzugt bei einem pH-Wert unterhalb von 4 mit Esterbindungen. Für die Ausbildung von Esterbindungen können in die Stärkepolymere Carboxyalkylgruppen eingeführt werden. Besonders bevorzugt sind Carboxymethylhydroxyethylstärken, mit einem DS für Carboxymethylgruppen von 0,03 bis 0,1 und einem DS für Hydroxyethylgruppen von 0,2 bis 0,3 und einem Molekulargewicht von 30.000 bis 300.000. Bei sehr kleinen Heparinmolekülen mit 1 bis 4 Saccharideinheiten eine Bindung an das Polysaccharid erfolgen, die das lineare Heparinmolekül von dem Polysaccharid frei abstehen lässt.

In einer besonderen Ausgestaltung der vorliegenden Erfindung werden durch reduktive Aminierung Aminogruppen in eine Hydroxyalkylstärke oder Carboxyalkylstärke eingeführt. Mit den eingeführten Aminogruppen des Polysaccharids können beispielsweise die terminalen Aldehydgruppen des Heparins so eingeführt werden, dass der Rest des Heparinmoleküls frei bleibt. Die durch reduktive Aminierung eingeführten Aminogruppen werden auch für Reste mit Carboxylgruppen, terminalen Aldehydgruppen, Carbonsäurehalogeniden, Carboxyalkylen oder Estern zur kovalenten Bindung genutzt.

Die reduktive Aminierung der alpha-1,4-glycosidisch verknüpfte Polysaccharide T1 und/oder T2 erfolgt vorteilhaft mit Ammoniak, Alkylaminen, Dialkylaminen oder Ammoniumhydroxid in Anwesenheit eines Reduktionskatalysators. Die Reduktion erfolgt bevorzugt in einer Wasserstoffatmosphäre unter erhöhtem Druck und Temperaturbedingungen. Als Katalysatoren kommen beispielsweise Raney Nickel, Cobalt Nickel Katalysatoren und/oder Ruthenium Katalysatoren zum Einsatz. Der bei der reduktiven Aminierung mit Wasserstoff angewendete Druck und Temperaturen bewegen sich in dem Bereich von 80 bis 250 °C, vorzugsweise 100 bis 200 °C und Drücken von 2 bis 50 bar, vorzugsweise 5 bis 20 bar. Die aminierte Polyalkylstärke, beispielsweise Hydroxyalkylstärke, kann mit den Aldehydgruppen von Arzneistoffen Heparin unter Bildung eines Imins umgesetzt werden. Im nächsten Schritt wird das Imin zum Amin reduziert. Die Aminogruppe des aminierten Polysaccharids reagiert dann mit der Aldehydgruppe des Arzneimittelwirkstoffs unter Ausbildung einer Schiff'schen-Base. Diese wird durch ein geeignetes Reduktionsmittel ausgesucht aus der Gruppe der salzhaltigen Hydride, Lithiumaluminiumhydrid, Lithiumborhydrid, Natriumborhydrid oder Natriumcyanoborhydrid zum Amin reduziert. Dabei muss berücksichtigt werden, dass auch die aminierten Polysaccharide, beispielsweise Hydroxyalkylstärken jeweils eine terminale Aldehydgruppe aufweisen. Die Verwendung aminierter Polysaccharide ermöglicht ein weiteres Verfahren in zwei Schritten. Dabei wird das zum Einbau vorgesehene Glucosaminoglucan in einem ersten separaten Schritt zum Lacton oxidiert, welches in einem weiteren Schritt mit der Aminogruppe der aminierten Hydroxyalkylstärke unter Bildung eines Carbonsäureamids verbunden wird. Bevorzugt wird das Verfahren nach Hashimoto (Hashimoto et al., Kunststoffe, Kautschuk, Fasern, Vol. 9 (1992), Seite 1271 bis 1279) verwendet.

In einer besonders bevorzugten Ausgestaltung können die Aminogruppen der Polysaccharide T1 und/oder T2 zur kovalenten Ausbildung, insbesondere von Heparinen verwendet werden.

Die Erfindung wird durch die nachfolgenden Beispiele näher erläutert.

### Beispiele

### Beispiel 1

200 g einer Hydroxyethylstärke mit einem Molekulargewicht von 50.000 und einer molaren Substitution von 0,3 wird zusammen mit einer 27%igen Ammoniumhydroxidlösung zusammen mit 400 g eines Nickel/Kupfer/Chrom Katalysators mit einem Nickelanteil von 75%, einem Kupferanteil von 23% und einem Chromanteil von 2% in einen Autoclaven eingebracht. Unter Zugabe von Wasserstoff wird über einen Zeitraum von 12 Stunden stufenweise mit den Schritten 100 bar, 150 bar, 170 bar beaufschlagt. Vor jeder Druckerhöhung wird eine Probe entnommen, dialysiert und gefriergetrocknet.

Die Temperatur wird bis auf 220 °C erhöht. Anschließend wird das Gemisch entnommen, dialysiert und gefriergetrocknet. 200 mg Heparin werden in 5 ml PBS (phosphate buffered soline; phosphatgepufferte Salzlösung); pH = 7,5 gelöst und in ein Reaktionsgefäß pipettiert. 200 mg der reduktiv aminierten Hydroxyethylstärke werden in 10 ml destilliertem Wasser gelöst und vorsichtig zugegeben. Danach werden 0,025 mg Natriumcyanoborhydrid (NaBCNH₃) werden zugemischt. Das Petriglas wird vorsichtig geschüttelt. Nach 2 Stunden werden erneut 0,025 mg des Natriumcyanoborhydrids zugegeben und vorsichtig solange geschüttelt, bis keine Bläschen mehr aufsteigen. Die Zugabe von Natriumborhydrid wird 4mal in gleicher Weise wiederholt. Danach wird das Reagenz über 72 Stunden stehen gelassen; schließlich in einem Überschuss PBS pH = 7,5 aufgenommen, dialysiert und gefriergetrocknet.

200 mg des Reagenzes werden in 200 ml destilliertem Wasser gelöst. Das Gemisch wird durch Zugabe einer 1N NaOH /Acetonlösung (30/70) auf pH 10 eingestellt und geschüttelt. In das Gefäß werden 0,2 ml 1,2,7,8-Diepoxyoctan pipettiert und geschüttelt. Die Zugabe von 0,2 ml 1,2,7,8-Diepoxyoctan wird alle 10 Stunden wiederholt. Nach 46 Stunden wird die Lösung entnommen gegen destilliertes Wasser dialysiert und gefriergetrocknet. Das Reagenz wird in 10 ml PBS; pH = 7,5 aufgenommen

### Beispiel 2

200 g einer Hydroxyethylstärke mit einem Molekulargewicht von 50.000 und einer molaren Substitution von 0,4 werden zusammen in einer 27%igen Ammoniumhydroxidlösung gelöst.

Die Lösung wird mit 400 g eines Nickel/Kupfer/Chrom Katalysators in einen Autoklaven eingebracht. Unter Zugabe von Wasserstoff wird über einen Zeitraum von 12 Stunden stufenweise mit den Schritten 100 bar, 150 bar und 170 bar beaufschlagt. Vor jeder Druckerhöhung wird eine Probe entnommen, dialysiert und gefriergetrocknet. Die Temperatur wird auf 270 °C erhöht. Anschließend wird das Gemisch entnommen, dialysiert und gefriergetrocknet. Die entnommenen Proben werden mit 200 mg Heparin in 5 ml PBS (pH = 7,5) gelöst. 200 mg der reduktiv aminierten Hydroxyethylstärke werden in 10 ml destilliertem Wasser gelöst und vorsichtig zugegeben. Danach werden 0,025 mg Natriumcyanoborhydrid (NaBCNH₃) zugemischt. Die Petrischale wird vorsichtig geschüttelt. Nach 2 Stunden werden erneut 0,025 mg des Natriumcyanoborhydrids zugegeben und vorsichtig solange geschüttelt, bis keine Bläschen mehr aufsteigen. Die Zugabe von Natriumborhydrid wird 4mal in gleicher Weise wiederholt. Danach wird das Reagenz über 24 Stunden stehen gelassen. Das Reagenz wird schließlich in einem Überschuss PBS (pH = 7,5) aufgenommen, dialysiert und gefriergetrocknet.

200 mg einer Carboxymethylstärke mit einem DS von 0,4 werden mit dem Reagenz in 200 ml gelöst. Das Gemisch wird durch Zugabe einer 1N NaOH /Acetonlösung (30/70) gelöst auf einen pH von 10 eingestellt und geschüttelt. In das Gefäß werden 0,4 ml 1,2,7,8-Diepoxyoctan pipettiert und geschüttelt. Nach 12 Stunden wird die Lösung entnommen gegen destilliertes Wasser dialysiert und gefriergetrocknet. Das Reagenz wird in 10 ml PBS (pH = 7,5) aufgenommen.

### Beispiel 3

Verknüpfung einer aminierten Hydroxyethylstärke mit fluoreszenzmarkiertem Heparin über eine reduktive Aminierung und Verknüpfung der Reaktionsprodukte miteinander über eine weitere reduktive Aminierung mit Glutaraldehyd.
a) Kopplung von Heparin (Hep) mit dem Fluoreszenzmarker 2-Aminopyridin Zu einer Lösung aus 2-Aminoyridin (31.7 g, 0.33 Mol, 1000 Äqu.) und NaCNBH₃ (2.1g, 0.033 Mol, 100 Äqu.) in Formamid (50 ml) wird Heparin (5.0g) gegeben. Die erhaltene Suspension rührt man bei 37 °C über Nacht, wobei langsam eine klare Lösung entsteht. Die Reaktionslösung wird auf EtOH (50 ml) gegossen. Der ausgefallene Feststoff wird filtriert und getrocknet. Man erhält fluoreszenzmarkiertes Heparin (HEP*) als leicht beigen Feststoff (1,3 g).
b) Aminierung der Hydroxyethylstärke (HES)

   HES40 ----> HES40--NH₂

   HES 40 (5,1 g/MW 40 kDa) wird in einer wässrigen Ammoniumhydroxid-Lösung (100 ml, 22%ig) gelöst. Der Katalysator bestehend aus Nickel (5,6 g, 325 mesh, Chrom (0,15 g, 100 mesh) und Kupfer (1,8 g, 1 micron) wird zur Lösung zugegeben. Das Gemisch wird unter einer Wasserstoffatmosphäre bei 120 °C im Autoklav 48 h gerührt. Nach dem Abkühlen auf 20 °C filtriert man den Katalysator ab und gießt das Filtrat auf Ethanol (20 ml). Der ausgefallene Niederschlag wird filtriert, mit wenig Ethanol/ Wasser gewaschen und getrocknet. Man erhält die aminierte HES als leicht bläulichen Feststoff (1,2 g).
c) Reduktive Aminierung der in Schritt b) erhaltenen HES mit dem in Schritt a) erhaltenen fluoreszenzmarkierten Heparin (HEP*)
   HEP* (200 mg) wird in einer wässrigen Phosphatpuffer-Lösung (5 ml, pH = 7,5) gelöst und eine Lösung der aminierten Hydroxyethylstärke aus Schritt b) (200 mg) in destilliertem Wasser (10ml) zugetropft. Zur Reaktionslösung gibt man in Abständen von 2h sechsmal NaCNBH₃ (jeweils 0,025 mg, aus einer wässrigen Stammlösung). Das Reaktionsgemisch wird nochmals 2 h bei 20 °C gerührt. Zur weiteren Reinigung wird das Rohprodukt 24h dialysiert. Nach dem Eindampfen des Wassers wird das im Reaktionsschema dargestellte Verknüpfungsprodukt aus aminierter HES und fluoreszenzmarkiertem Heparin als farbloser Feststoff erhalten. Die Verbindung zeigt in wässriger Lösung als auch als Feststoff eine intensive grün-gelbe Fluoreszenz bei Bestrahlung mit UV-Licht mit 366 nm.
d) Reduktive Aminierung von mehreren in den Schritte a), b) und c) erhaltenen fluroreszenzmarkierten Heparin-Hydroxyethylstärkemolekülen mit Glutaraldehyd.

Die in den Schritten a), b) und c) erhaltene fluroreszenzmarkierte Heparin-Hydroxyethylstärke (0,5 mg) wird in einer wässrigen Phosphatpufferlösung (0,25 ml, pH=7,5) gelöst und mit Glutaraldehyd (0,25 ml, 25 Gew%) bei 20° gemischt. Zu der Reaktionslösung wird im Abstand von 2 h 3malig NaCNBH₃ (jeweils 0,01 mg) zugegeben und durch Schütteln gelöst. Das Gemisch wird über Nacht stehen gelassen. Es entsteht ein beigefarbenes Präzipitat. Das Reaktionsprodukt wird mit Ethylalkohol ausgefällt und eingedampft. Der Feststoff zeigt eine grün-gelbe Fluoreszenz bei Bestrahlung mit UV-Licht mit 366 nm.

### Beispiel 4

Verknüpfung einer aminierten Hydroxyethylstärke mit fluoreszenzmarkiertem Heparin und Hyaluronsäure über eine reduktive Aminierung und Verknüpfung der Reaktionsprodukte miteinander über eine weitere reduktive Aminierung mit Glutaraldehyd.

Hyaluronsäure (2 mg) wird in einer wässrigen Phosphatpuffer-Lösung (1,5 ml, pH = 7,5) gelöst und dem in Wasser gelösten Reaktionsprodukt aus den Schritten a), b) und c) aus Beispiel 3 zugemischt. Zu der Reaktionslösung wird im Abstand von 2 h 2malig NaCNBH₃ (jeweils 0,01 mg) zugegeben und durch Schütteln gelöst. Das Gemisch wird über Nacht stehengelassen.

Danach wird Glutaraldehyd (0,25 ml, 25 Gew%) bei 20 °C zugemischt. Zu der Reaktionslösung wird im Abstand von 2 h zweimalig NaCNBH₃ (jeweils 0,01 mg) zugegeben und durch Schütteln gelöst. Das Gemisch wird über Nacht stehen gelassen, dialysiert und gefriergetrocknet.

### Beispiel 5

Verknüpfung einer aminierten HES mit CMS 2 Gew.-Teile der wie in Beispiel 3 reduktiv aminierten HES werden mit 1 Gew.-Teil (Carboxymethylstärke) CMS, MW 100 kg/Dalton in wässriger Phosphatpufferlösung (pH 7,5) gelöst. Zur Reaktionslösung gibt man unter Rühren im Abstand von 2 Stunden 4mal NaCNBH₃ (0,025 mg) bis sich ein beiger Feststoff bildet. Das Reaktionsprodukt wird eingedampft.

In den Beispielen eingesetzte Reagenzien:
Heparin, Natrium-Salz (vom Schwein, porcine origin), pH =7, mittleres MW = 12-15 kDa, Hersteller: Changzou Qianhong Bio-Pharma Co., Ltd., Jiangsu, China.
HES40: Hydroxyethylstärke mit mittlerem Molekulargewicht MW= 40 kDa, Substitutionsgrad DS = 0,3; Hersteller BBraun, Crissier, Schweiz.
Glutardialdehyd (25 Gew%), Acros Organics, New Jersey, USA Heparin, Natrium-Salz (vom Schwein, porcine origin), pH = 7, mittleres MW = 12-15 kDa, Hersteller: Changzou Qianhong Bio-Pharma Co., Ltd., Jiangsu, China.
Natriumcyanoborhydrid, NaCNBH₃, Acros Organics, New Jersey, USA
Hyaluronsäure aus Streptococcus equi Alfa Aesar, Ward Hill, Massachusetts, USA

## Patentansprüche

1. Verknüpfungsprodukt umfassend mindestens die Polysaccharide T1 und T2, **dadurch gekennzeichnet, dass**
a) die Monosaccharide, aus denen die Polysaccharide T1 und T2 aufgebaut sind, vollständig alpha-1,4-glycosidisch miteinander verknüpft sind und
b) mindestens eines der Polysaccharide T1 und/oder T2 wenigstens eine Aminogruppe aufweist und
c) T1 und T2 durch mindestens einen Linker Z miteinander chemisch kovalent verbunden sind und
d) T1 und/oder T2 m Gruppen -(L-A) trägt, wobei
- A ein Arzneimittelwirkstoff und/oder ein Fluoreszenzmarker,
- L ein zweiter Linker, durch den T1 und/oder T2 mit A kovalent verknüpft ist, und
- m eine ganze Zahl, die mindestens 1 ist,
**dadurch gekennzeichnet, dass** die Polysaccharide T1 und T2 unabhängig voneinander ausgewählt sind aus der Gruppe, bestehend aus aminierter Hydroxyethylstärke, aminierter Carboxymethylstärke, aminierter Carboxyethylstärke, aminierter Hydroxyethyl-Carboxymethylstärke und aminierter Hydroxyalkylstärke, weiterhin **dadurch gekennzeichnet, dass** der Arzneimittelwirkstoff A Heparin ist, wobei das Verknüpfungsprodukt erhältlich ist durch reduktive Aminierung eines freie Aminogruppen (-NH₂) aufweisenden Polysaccharids T1 mit einem Polysaccharids T2, das mindestens eine Aldehyd- oder Ketogruppe aufweist und wobei das Polysaccharid T1 und/oder das Polysaccharid T2 mit m Resten -(L-A) verknüpft ist.

2. Verknüpfungsprodukt gemäß Anspruch 1, **dadurch gekennzeichnet, dass** die Polysaccharide T1 und/oder T2 Aminogruppen aufweisen, die durch reduktive Aminierung eingeführt wurden.

3. Verknüpfungsprodukt gemäß mindestens einem der Ansprüche 1 bis 2, **dadurch gekennzeichnet, dass** die Polysaccharide T1 und/oder T2 ein mittleres Molekulargewicht von 20000 bis 800000 Dalton, vorzugsweise 25000 bis 500000 Dalton, insbesondere von 30000 bis 200000 Dalton aufweisen.

4. Verknüpfungsprodukt gemäß mindestens einem der Ansprüche 1 bis 3, **dadurch gekennzeichnet, dass** die Polysaccharide T1 und/oder T2 Hydroxyethylstärke, mit einem Substitutionsgrad DS zwischen 0,2 und 0,8, vorzugsweise zwischen 0,3 und 0,6 aufweist.

5. Verknüpfungsprodukt gemäß mindestens einem der Ansprüche 1 bis 4, **dadurch gekennzeichnet, dass** der Fluoreszenzmarker ausgewählt ist aus der Gruppe bestehend aus Fluoresceinisothiocyanat (FITC), Phycoerythrin, Rhodamid und 2- Aminopyridin.

6. Verknüpfungsprodukt gemäß Anspruch 1, **dadurch gekennzeichnet, dass** A Heparin und das Polysaccharid T1 und/oder T2 eine Hydroxyethylstärke ist und der Linker L eine -NH-Gruppe darstellt.

7. Verknüpfungsprodukt gemäß mindestens einem der Ansprüche 1 bis 6, **dadurch gekennzeichnet, dass** die Polysaccharide T1 und/oder T2 bei 20 °C wasserlöslich sind.

8. Verknüpfungsprodukt gemäß mindestens einem der vorangehenden Ansprüche, **dadurch gekennzeichnet, dass** es als Flüssigkeit, Hydrogel, Film oder als Feststoff vorliegt.

9. Pharmazeutische Zubereitung, umfassend das Verknüpfungsprodukt gemäß mindestens einem der Ansprüche 1 bis 8.

10. Pharmazeutische Zubereitung gemäß Anspruch 9 zur Verwendung in der Prophylaxe von Verwachsungen und Narbenbildung oder zur Verwendung bei der Stillung von Blutungen oder als Synovialflüssigkeit oder in der Therapie und Prophylaxe der Wundheilung.

11. Verknüpfungsprodukt gemäß mindestens einem der Ansprüche 1 bis 8 zur Wundabdeckung oder als Implantat.

12. Verfahren zur Herstellung eines Verknüpfungsprodukts gemäß Anspruch 1, durch Verknüpfen mindestens eines Polysaccharids T1 mit mindestens einem Polysaccharid T2 unter Ausbildung des Linkers Z mit dem T1 und T2 kovalent miteinander verknüpft sind und wobei T1 und/oder T2 m Gruppen-(L-A) trägt, wobei- A Heparin ist,
- L ein zweiter Linker, durch den T1 und/oder T2 mit A kovalent verknüpft ist und
- m eine ganze Zahl ist, die mindestens 1 ist, wobei die Polysaccharide T1 und/oder T2 zunächst mit dem Arzneimittelwirkstoff A verknüpft und anschließend die Ausbildung des Linkers Z durchgeführt wird.

## Claims

1. Bonding product comprising at least the polysaccharides T1 and T2,
**characterized in that**
a) the monosaccharides from which the polysaccharides T1 and T2 are composed are completely alpha-1,4-glycosidically linked to each other and
b) at least one of the polysaccharides T1 and/or T2 has at least one amino group and
c) T1 and T2 are chemically covalently bound to each other by at least one linker Z and
d) T1 and/or T2 carries m groups -(L-A), wherein
- A is an active pharmaceutical ingredient and/or a fluorescent marker,
- L is a second linker by which T1 and/or T2 is covalently linked to A, and
- m is an integer that is at least 1,
**characterized in that** the polysaccharides T1 and T2 are independently selected from the group consisting of aminated hydroxyethyl starch, aminated carboxymethyl starch, aminated carboxyethyl starch, aminated hydroxyethyl-carboxymethyl starch and aminated hydroxyalkyl starch, further **characterized in that** the active pharmaceutical ingredient A is heparin, the bonding product being obtainable by reductive amination of a polysaccharide T1 having free amino groups (-NH₂) with a polysaccharide T2 having at least one aldehyde or keto group and wherein the polysaccharide T1 and/or the polysaccharide T2 is linked to m residues -(L-A).

2. Bonding product according to claim 1, **characterized in that** the polysaccharides T1 and/or T2 have amino groups introduced by reductive amination.

3. Bonding product according to at least one of claims 1 to 2,
**characterized in that** the polysaccharides T1 and/or T2 have an average molecular weight of 20,000 to 800,000 daltons, preferably 25,000 to 500,000 daltons, in particular 30,000 to 200,000 daltons.

4. Bonding product according to at least one of claims 1 to 3, **characterized in that** the polysaccharides T1 and/or T2 have hydroxyethyl starch, with a degree of substitution DS of between 0.2 and 0.8, preferably between 0.3 and 0.6.

5. Bonding product according to at least one of claims 1 to 4, **characterized in that** the fluorescent marker is selected from the group consisting of fluorescein isothiocyanate (FITC), phycoerythrin, rhodamide and 2-aminopyridine.

6. Bonding product according to claim 1, **characterized in that** A heparin and the polysaccharide T1 and/or T2 is a hydroxyethyl starch and the linker L represents an -NH-group.

7. Bonding product according to at least one of claims 1 to 6, **characterized in that** the polysaccharides T1 and/or T2 are water-soluble at 20°C.

8. Bonding product according to at least one of the preceding claims, **characterized in that** it is present as liquid, hydrogel, film or solid.

9. Pharmaceutical preparation comprising the bonding product of at least one of claims 1 to 8.

10. Pharmaceutical preparation according to claim 9 for use in the prophylaxis of adhesions and scar formation or for use in stopping bleeding or as synovial fluid or in the therapy and prophylaxis of wound healing.

11. Bonding product according to at least one of claims 1 to 8 for wound coverage or as an implant.

12. Process for the preparation of a bonding product according to claim 1, by binding at least one polysaccharide T1 with at least one polysaccharide T2 to form the linker Z with which T1 and T2 are covalently linked to one another, and wherein T1 and/or T2 carries m groups-(L-A), wherein- A is heparin,
- L is a second linker by which T1 and/or T2 is covalently linked to A and
- m is an integer which is at least 1, wherein the polysaccharides T1 and/or T2 are first linked to the active pharmaceutical ingredient A and then the formation of the linker Z is carried out.

## Revendications

1. Produit de liaison comprenant au moins les polysaccharides T1 et T2, **caractérisé en ce que**
a) les monosaccharides, à partir desquels sont composés les polysaccharides T1 et T2, sont liés entre eux totalement par une liaison alpha-1,4-glycosidique et
b) au moins l'un des polysaccharides T1 et/ou T2 présente au moins un groupe amino et
c) T1 et T2 sont liés entre eux par une liaison chimique covalente au moyen d'au moins un lieur Z et
d) T1 et/ou T2 portent m groupes -(L-A),
- A étant un principe actif pharmaceutique et/ou un marqueur fluorescent,
- L étant un second lieur, au moyen duquel T1 et/ou T2 est lié à A par covalence, et
- m étant un nombre entier, valant au moins 1,
**caractérisé en ce que** les polysaccharides T1 et T2 sont choisis indépendamment l'un de l'autre parmi le groupe se composant de l'amidon hydroxyéthylé aminé, l'amidon carboxyméthylé aminé, l'amidon carboxyléthylé aminé, l'amidon carboxyméthylé-hydroxyéthylé aminé et l'amidon hydroxyalkyle aminé, **caractérisé en outre en ce que** le principe actif pharmaceutique A est l'héparine, le produit de liaison pouvant être obtenu par amination réductrice d'un polysaccharide T1 présentant un groupe amino libre (-NH₂) avec un polysaccharide T2, qui présente au moins un groupe aldéhyde ou cétone et le polysaccharide T1 et/ou le polysaccharide T2 est lié aux m radicaux -(L-A).

2. Produit de liaison selon la revendication 1, **caractérisé en ce que** les polysaccharides T1 et/ou T2 présentent des groupes amino, qui ont été introduits par amination réductrice.

3. Produit de liaison selon au moins l'une quelconque des revendications 1 à 2, **caractérisé en ce que** les polysaccharides T1 et/ou T2 présentent une masse moléculaire moyenne allant de 20 000 à 800 000 Daltons, de préférence de 25 000 à 500 000 Daltons, en particulier de 30 000 à 200 000 Daltons.

4. Produit de liaison selon au moins l'une quelconque des revendications 1 à 3, **caractérisé en ce que** les polysaccharides T1 et/ou T2 présentent un amidon hydroxyéthylé, comportant un degré de substitution DS compris entre 0,2 et 0,8, de préférence entre 0,3 et 0,6.

5. Produit de liaison selon au moins l'une quelconque des revendications 1 à 4, **caractérisé en ce que** le marqueur fluorescent est choisi parmi le groupe se composant de l'isothiocyanate de fluorescéine (FITC), la phycoérythrine, la rhodamine et la 2-aminopyridine.

6. Produit de liaison selon la revendication 1, **caractérisé en ce que** A est l'héparine et le polysaccharide T1 et/ou T2 est un amidon hydroxyéthylé et **en ce que** le lieur L représente un groupe -NH-.

7. Produit de liaison selon au moins l'une quelconque des revendications 1 à 6, **caractérisé en ce que** les polysaccharides T1 et/ou T2 sont solubles dans l'eau à 20 °C.

8. Produit de liaison selon au moins l'une quelconque des revendications précédentes, **caractérisé en ce qu'**il est présent sous forme liquide, d'hydrogel, d'un film ou sous forme solide.

9. Composition pharmaceutique, comprenant le produit de liaison selon au moins l'une quelconque des revendications 1 à 8.

10. Composition pharmaceutique selon la revendication 9 destinée à être utilisée en prophylaxie des adhérences et de la formation de cicatrices ou destinée à être utilisée pour arrêter les hémorragies ou en tant que liquide synovial ou dans le traitement et en prophylaxie de la cicatrisation des plaies.

11. Produit de liaison selon au moins l'une quelconque des revendications 1 à 8 pour recouvrir une plaie ou en tant qu'implant.

12. Procédé de fabrication d'un produit de liaison selon la revendication 1, par liaison d'au moins un polysaccharide T1 à au moins un polysaccharide T2 en formant le lieur Z avec lequel T1 et T2 sont liés entre eux par covalence et T1 et/ou T2 porte m groupes -(LA), A étant l'héparine,
- L étant un second lieur, au moyen duquel T1 et/ou T2 est lié par covalence à A et
- m est un nombre entier, qui vaut au moins 1, les polysaccharides T1 et/ou T2 étant liés d'abord avec le principe actif pharmaceutique A, puis la formation du lieur Z étant réalisée.
